Europäisches Patentamt

European Patent Office

Office européen des brevets

(19)

(11) **EP 0 719 286 B1**

(12) **EUROPÄISCHE PATENTSCHRIFT**

(45) Veröffentlichungstag und Bekanntmachung des Hinweises auf die Patenterteilung:
**03.12.1997 Patentblatt 1997/49**

(51) Int Cl.⁶: **C08B 37/00**, A61K 9/16, A61K 9/20, A61K 9/28, A61K 9/50

(21) Anmeldenummer: **94926923.7**

(22) Anmeldetag: **07.09.1994**

(86) Internationale Anmeldenummer:
**PCT/EP94/02988**

(87) Internationale Veröffentlichungsnummer:
**WO 95/07936 (23.03.1995 Gazette 1995/13)**

(54) **DEXTRANESTER, VERFAHREN ZU IHRER HERSTELLUNG UND IHRE VERWENDUNG ZUR UMHÜLLUNG ODER EINBETTUNG VON ARZNEIMITTELN**

DEXTRAN ESTERS, PROCESS FOR PRODUCING THEM AND THEIR USE FOR ENCASING AND ENCAPSULATING MEDICAMENTS

ESTERS DE DEXTRANE, LEUR PROCEDE DE PREPARATION ET LEUR UTILISATION POUR ENROBER OU ENCAPSULER DES MEDICAMENTS

(84) Benannte Vertragsstaaten:
**AT BE CH DE FR GB IT LI NL SE**

(30) Priorität: **17.09.1993 DE 4331539**

(43) Veröffentlichungstag der Anmeldung:
**03.07.1996 Patentblatt 1996/27**

(73) Patentinhaber: **BASF AKTIENGESELLSCHAFT**
**67056 Ludwigshafen (DE)**

(72) Erfinder:
- **SCHEHLMANN, Volker**
  **D-67354 Römerberg (DE)**
- **BAUER, Kurt, Heinz**
  **D-79112 Freiburg (DE)**
- **KESSELHUT, Jan-Frederic**
  **D-79114 Freiburg (DE)**

(56) Entgegenhaltungen:
EP-A- 0 095 968           WO-A-92/00732
WO-A-92/13006             DE-A- 4 136 324
US-A- 2 954 372

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft wasserunlösliche Dextranester, ein Verfahren zu ihrer Herstellung, ihre Verwendung zur Umhüllung und/oder Einbettung von Arzneiwirkstoffen oder Arzneimittelzubereitungen sowie Arzneimittel, enthaltend einen Wirkstoff, umhüllt von oder eingebettet in einen Dextranester.

Bei der Entwicklung und Formulierung moderner Arzneimittel kommen Hilfsstoffen heutzutage eine immer größere Bedeutung zu. So ist nicht nur der Arzneistoff alleine verantwortlich für eine gezielte Wirkung, sondern vielmehr das Zusammenspiel mit einem oder mehreren zugesetzten Hilfsstoffen. Besondere Bedeutung kommt den Hilfsstoffen bei der zeitlichen oder ortsspezifischen Arzneistoffliberation sowie der Resorption zu. Bei der Verabreichung von peroralen Arzneiformen ist bisher eine zeitliche oder ortsspezifische Arzneistoffreisetzung auf den Magen und verschiedene Bereiche des Dünndarms, durch die Wahl geeigneter Umhüllungsmaterialien, beschränkt. Es gibt bis heute jedoch keine geeigneten Umhüllungsmaterialien mit deren Hilfe es gelingt, die Arzneiform unverändert und voll wirksam bis in den Dickdarm zu transportieren, um dort den Arzneistoff gezielt freizusetzen. Eine solche Arzneiform wäre beispielsweise wünschenswert bei der lokalen Therapie von entzündlichen Erkrankungen der Dickdarmschleimhaut, wie z.B. Morbus Crohn. Außerdem könnten neue Wege bei der Behandlung mit Peptidarzneistoffen beschrieben werden, die bei der peroralen Applikation durch physiologische bzw. enzymatische Einflüsse der Magen- und Dünndarmsäfte verdaut und damit unwirksam werden. Bei der Entwicklung einer Peptidschluckarzneiform ergibt sich gegenüber anderen möglichen Applikationswegen, wie die nasale, transdermale oder pulmonale Verabreichung, der Vorteil, daß keine weiteren Hilfsmittel (z.B. Pflaster, Zerstäuber) notwendig sind. Dies führt zu verminderten Therapiekosten, sowie zu einer erhöhten Compliance, da die perorale Arzneistoffgabe vom Patienten als natürlicher empfunden wird und außerdem von ihm selbst durchgeführt werden kann.

Für eine gezielte Arzneistoffreisetzung im menschlichen Dickdarm müssen an ein Umhüllungsmaterial eine Reihe von Anforderungen gestellt werden:

1. Das Einbettungs- bzw. Umhüllungsmaterial muß wasserunlöslich und durch bakterielle Enzyme im Dickdarm gespalten werden.

2. Es sollte gerade noch ausreichende Wasserquellung besitzen, da dies für einen enzymatischen Angriff erforderlich ist.

3. Es muß magensaft- und dünndarmsaftresistent sein.

4. Das Material und seine Abbauprodukte sollten untoxisch und physiologisch verträglich sein.

Es besteht ein deutlicher Unterschied in der Dichte der Bakterienbesiedelung zwischen Dünndarm ($10^4$ Keime/ml) und Dickdarm ($10^{14}$ Keime/ml). Deshalb kann die enzymatische Aktivität einiger Bakterien der Dickdarmflora für die Spaltung eines dünndarmstabilen Filmüberzugs herangezogen werden.

Aus J. Chem. Soc., 1952, 74, 5016 sind Stearyldextrane bekannt. Doch ist dort weder eine Angabe zur Molmasse noch zum Substituti-onsgrad (Veresterungsgrad) zu finden. Dementsprechend ist auch keine Lehre gegeben, wie diese Parameter eingestellt werden müssen, damit die Substanzen von Dickdarmbakterien abgebaut werden.

In der DE 40 06 521 A1 (und der ihre entsprechenden europäischen Pacentanmeldung 450 176 A1) werden zukkerhaltige Polymere zur Umhüllung und Einbettung von Arzneistoffen beschrieben. Diese zukkerhaltigen Polymere werden zur Umhüllung und/oder Einbettung von oral applizierbaren pharmazeutischen Wirkstoffen verwendet und bewirken, daß die Wirkstoffe, die in den Polymeren enthalten sind, erst im Dickdarm freigesetzt werden. Die in dieser Druckschrift beschriebenen Polymere besitzen den Nachteil, daß sie kompliziert hergestellt werden müssen und mit Polyisocyanaten vernetzt sind.

Aus der DE 41 36 324 A1 sind Dextranderivate bekannt, die ein Molekulargewicht bis 20 000 aufweisen und als Adsorptionsmittel für Gallensäuren vorgesehen sind.

Aus der DE 41 31 292 A1 sind Galaktomannanderivate zur Umhüllung oder Einbettung von Arzneimitteln bekannt, wobei die Galaktomannane verethert oder verestert werden. Das Herstellungsverfahren und die Reinigung der veretherten bzw. veresterten Galaktomannan- derivate ist jedoch kompliziert und aufwending.

Der vorliegenden Erfindung lag daher die Aufgabe zugrunde, Hilfsstoffe für Arzneimittel zur Verfügung zu stellen, welche die oben genannten Anforderungen für dickdarmabbaubare Materialien erfüllen, sich von leicht zugänglichen Ausgangsstoffen ableiten und verfahrenstechnisch ohne Schwierigkeiten hergestellt sowie verarbeitet werden können.

Diese Aufgabe wird gelöst durch Dextranester mit einer Molmasse von 40 000 bis 10 000 000 und Esterseitenketten, die sich von Säuren mit 6 bis 18 C-Atomen ableiten, wobei der Veresterungsgrad in Abhängigkeit von der C-Atomzahl der Seitenketten und der Molmasse auf einen Wert von 0,04 bis 1,1 so eingestellt wird, daß die Dextranester bei Raumtemperatur in Wasser unlöslich sind und von Dickdarmbakterien abgebaut werden.

Es hat sich überraschenderweise gezeigt, daß ausgehend von Dextranen Einbettungsmaterialien hergestellt werden können, welche die obengenannten Voraussetzungen erfüllen. Ein besonderer Vorteil ergibt sich dann, wenn die erfindungsgemäßen Dextranester zusätzlich filmbildende Eigenschaften aufweisen, da sie in diesem Fall nicht nur als Einbettungsmaterialien, sondern auch als Umhüllungsmaterialien geeignet sind.

In diesem Zusammenhang ist es von besonderer Bedeutung, daß die erfindungsgemäßen Dextranester in umweltverträglichen Lösungsmittelgemischen wie Wasser/Alkohol-Gemischen löslich oder zumindest dispergierbar sind, da sich dadurch erweiterte Möglichkeiten ergeben, die Dextranester als Filme aufzubringen.

Die Erfindung betrifft demnach weiterhin die Verwendung der Dextranester zur Umhüllung und/oder Einbettung von Arzneiwirkstoffen oder Arzneimittelzubereitungen sowie Arzneimittel, die einen im Dickdarm wirkenden Wirkstoff oder einen Wirkstoff, der beim Durchgang durch den Magen oder Dünndarm abgebaut wird, umhüllt mit oder eingebettet in den erfindungsgemäßen Dextranestern enthalten.

Damit die erfindungsgemäßen Dextranester als Umhüllungsmaterialien geeignet sind, müssen verschiedene Faktoren berücksichtigt werden.

So stellen Molekülmasse und Substitutionsgrad für die Filmbildung einerseits und für die Abbaubarkeit andererseits gegenläufige Faktoren dar. Wird eine hohe Molekülmasse gewählt, ist die Filmbildung begünstigt, der Zerfall des Films nach enzymatischem Angriff ist jedoch verlangsamt. Wird ein hoher Substitutionsgrad durch Synthese erreicht, ist die Stabilität der Filme in Wasser verbessert, die Quellung und die Abbaubarkeit jedoch verringert.

Die Erfindung betrifft außerdem ein Verfahren zur Herstellung der Dextranester, bei dem Dextran in einem Lösungsmittel aus Formamid und/oder Dimethylsulfoxid, dem eine derartige Menge eines aprotischen, polaren, organischen Solven zugemischt werden kann, daß das Dextran noch löslich ist, gelöst wird und ein Halogenid, insbesondere ein Chlorid, einer Säure mit 6 bis 18 C-Atomen in Gegenwart eines Protonenfängers so zugegeben wird, daß die Temperatur des Reaktionsgemisches nicht über 40°C steigt. Als Protonenfänger werden Amine, insbesondere Pyridin, eingesetzt.

Die zur Herstellung der erfindungsgemäßen Dextranester eingesetzten Dextrane sind ohne weiteres zugänglich.

Sie werden z.B. aus Bakterienkulturen von Leuconostoc-Arten gewonnen. Je nach Leuconostoc-Art können Dextrane mit unterschiedlicher Struktur isoliert werden. Beispiele für einsetzbare Dextrane sind in der Literatur beschrieben (J. Am. Chem. Soc. 76, 5041, 1964). Der $\alpha$-1,6-Bindungsanteil in den Dextranen und damit in den resultierenden Dextranestern sollte jedoch 60 % nicht unterschreiten, da Dextrane mit einem $\alpha$-1,6-Bindungsanteil unter 60 % nur noch bedingt durch Dextranase enzymatisch abgebaut werden. Die Abbaubarkeit von Dextranen durch Darmbakterien ist im übrigen bekannt (J. of Bact., 63, 424, 1951).

Bei dem käuflich erhältlichen Dextran handelt es sich um z.B. um den Typ NRRL-512. Dieser wird aus Kulturen von Leuconostoc mesenteroides isoliert. Dextran NRRL-512 stellt ein $\alpha$-1,6-Polyglucan dar. Der Anteil an $\alpha$-1,6-Bindungen liegt bei ca. 95 %. Die restlichen Bindungen stellen $\alpha$-1,2 und $\alpha$-1,4-Verknüpfungen der Glucosemonomere dar, die zu Verzweigungen mit einer Kettenlänge von einer Glucoseeinheit führen. Dextran NRRL-512 stellt somit ein nahezu unverzweigtes Zuckerpolymer dar.

Dextrane sind in vielen Molmassenbereichen zwischen 800 und 10 000 000 erhältlich. Nach Isolierung des hochmolekularen Dextrans aus der Bakterienkultur, wird dieses sauer hydrolysiert und die verschiedenen Molmassenbereiche durch Fraktionierung mit Ethanol-Wasser-Gemischen unterschiedlicher Konzentrationen gewonnen.

Die Molmassen der Ausgangsdextrane für die erfindungsgemäßen Dextranester werden so gewählt, daß nach Derivatisierung Produkte mit den erforderlichen Abbau- und Löslichkeits- bzw. Quellungseigenschaften gewonnen werden können.

Dextran erfüllt einen Teil der eingangs erwähnten Anforderungen, die an einen dickdarmabbaubaren Überzug gestellt werden müssen. Es ist jedoch wasserlöslich und muß deshalb gezielt durch Substitution mit geeigneten Substituenten hydrophobisiert werden. Art und Anzahl der eingeführten Substituenten bestimmen entscheidend die Löslichkeit- bzw. Quellungseigenschaften, sowie Filmbildung und enzymatische Abbaubarkeit.

Es wurde gefunden, daß für einen enzymatischen Angriff im Dickdarm unsubstituierte Bereiche im Dextranester vorliegen müssen. Die erfindungsgemäßen Dextranester werden jedoch durch Amylasen, die im Magen und Dünndarm vorkommen, nicht angegriffen und sind daher dünndarmstabil.

Bevorzugt werden Dextranester, deren Esterseitenketten sich von Säuren mit 8 bis 16, insbesondere 8 bis 12 C-Atomen ableiten, deren Molmasse 40 000 bis 1 000 000, insbesondere 60 000 bis 400 000, beträgt und die einen Veresterungsgrad von 0,08 bis 0,8, insbesondere 0,1 bis 0,5 aufweisen.

Dabei sind folgende Dextranester besonders bevorzugt:

Für dickdarmabbaubare Filme und Einbettungen empfehlen sich Dextranester mit Lauroylsubstituenten und einem Substitutionsgrad (Veresterungsgrad) zwischen DS = 0,1 und 0,5, bevorzugt jedoch zwischen DS = 0,1 und 0,2. Die Molmasse der Lauroyldextrane soll dabei zwischen 150.000 und 1.000.000, bevorzugt jedoch zwischen 200.000 und 300.000 liegen.

Nur für Einbettungen dagegen sind Caproyldextrane zwischen DS = 0,1 und 0,5, bevorzugt jedoch zwischen DS = 0,1 und 0,2 und einer Molmasse zwischen 1.000.000 und 10.000.000 geeignet; außerdem auch Stearoyldextrane mit DS = 0,1-0,5, bevorzugt jedoch zwischen DS = 0,1 und 0,2 und einer Molmasse zwischen 150.000 und 1.000.000, bevorzugt jedoch zwischen 200.000 und 300.000; des weiteren Lauroyldextrane mit DS = 0,2-0,5 und einer Molmasse zwischen 60.000 und 150.000.

Die erfindungsgemäßen Dextranester können z.B. wie folgt hergestellt werden:

Dextran wird im Vakuumtrockenschrank getrocknet und in einem Rundkolben mit Calciumchloridtrockenrohr in einer Mischung aus Formamid und Pyridin gelöst. Anschließend wird das Fettsäurechlorid zugesetzt und der Reaktionsansatz vier Stunden bei Raumtemperatur gerührt. Das entstandene Reaktionsprodukt wird in Wasser gefällt, abgetrennt und mehrfach mit Wasser gewaschen. Schließlich wird noch mehrfach mit einem Gemisch aus Ethylacetat und Ethanol gewaschen und das Produkt getrocknet.

Aus J. Am. Chem. Soc., 1952, 74, 5339 ist ein vergleichbares Verfahren zur Herstellung von Dextrantriacetaten bekannt.

Zur Herstellung der erfindungsgemäßen Ester muß wasserfrei gearbeitet werden. Als Lösungsmittel kommen hier vor allem aprotische, organische, polare Solventien wie Formamid, N-Methylpyrrolidon, Dimethylformamid, Dimethylacetamid und/oder Dimethylsulfoxid in Frage. Die Veresterung kann auch ohne Lösungsmittel in der Schmelze durchgeführt werden. In diesem Falle dient als Lösungsmittel für das Polymer das Acylierungsreagens beziehungsweise der Reaktionspartner, zum Beispiel Alkaloylhalogenid, Alkaloylanhydrid oder Chloracetanhydrid. Die Reaktion der Veresterung erfolgt beispielsweise bei Temperaturen zwischen 0° bis 160°C, bzw. beim Siedepunkt des Lösungsmittels, durch Umsetzung der entsprechenden Dextrane mit $C_6$-$C_{18}$-Alkanoylhalogeniden, vorzugsweise $C_8$-$C_{16}$-Alkanoylhalogeniden oder $C_6$-$C_{18}$-Alkanoylanhydriden, vorzugsweise $C_8$-$C_{16}$-Alkanoylanhydriden.

Zweckmäßig erfolgt diese Acylierung in Gegenwart basischer Verbindungen, wie zum Beispiel Pyridin.

Die basischen Substanzen sollen bezogen auf die Ausgangsalkanoylverbindung im Überschuß vorliegen, beispielsweise im Überschuß von 0,1 bis 0,2 Mol pro Mol der Alkanoylausgangskomponente.

Insbesondere betrifft die Erfindung die Verwendung der erfindungsgemäßen Dextranester zur Herstellung von Filmüberzügen und Einbettungen von pharmazeutischen Wirkstoffen, insbesondere von oral-applizierbaren Wirkstoffen, bzw. oral-applizierbaren Arzneizubereitungen mit einer Wirkstoff-Freigabe im Dickdarm. Dies wird dadurch erreicht, daß die Wirkstoffe oder Zubereitungen mit Wirkstoffen, zum Beispiel Granulate, Pellets oder Tabletten, mit den erfindungsgemäßen Dextranestern umhüllt und/oder in diese eingebettet sind.

Die Umhüllung der Wirkstoffe oder der pharmazeutischen Zubereitungen, das heißt der Zubereitungen, worin die Wirkstoffe zusammen mit üblichen oder erforderlichen pharmazeutischen Hilfsstoffen eingearbeitet sind, erfolgt nach den in der pharmazeutischen Technologie bekannten Methoden, bzw. den üblichen Verfahren zum Überziehen von Arzneiformen. Das Einbetten von therapeutischen Wirkstoffen erfolgt ebenfalls nach in der pharmazeutischen Technologie bekannten Methoden. Hierbei können weiterhin übliche pharmazeutische Hilfs- bzw. Zusatzstoffe mitverwendet werden, beispielsweise Weichmacher (insbesondere bei Umhüllungen), Aromastoffe, Süßmittel, Hilfsstoffe wie zum Beispiel Talkum, Calciumcarbonat, Mannitol, Cellulosepulver, lösliche Farbstoffe und Pigmente.

Die einsetzbaren zusätzlichen Hilfsstoffe, ihre Verwendung und die Herstellung von Arzneimittelzubereitungen sind vielfach beschrieben, z.B. in der DE 41 31 292 A1, Spalten 5 bis 10, und dem Fachmann daher bekannt.

Als Wirkstoffe, die vorzugsweise mit den erfindungsgemäßen Dextranen formuliert werden können, kommen beispielsweise solche Arzneiwirkstoffe in Frage, die im Magen oder Dünndarm abgebaut oder verdaut werden und deshalb peroral in der Vergangenheit nicht appliziert werden konnten, und Arzneimittel, die erst im Dickdarm wirken sollen, wie Arzneimittel, die gegen Dickdarmkrankheiten wirken, und Peptidarzneimittel. Beispiele sind: Peptide, Herz-Kreislauf-Therapeutika, Antirheumatika/Analgetika, Mittel zur Therapie von Dickdarmerkrankungen wie Morbus Crohn und Colitis Ulcerosa, Antiasthmatika, Antifibrinolytika, Antihämorrhagika, Antitumormittel, Enzympräparate, Antibiotika, Antimykotika, Substanzen mit Wirkung auf das Zentralnervensystem.

Beispiele für Peptidwirkstoffe sind: ACTH (Adrenocorticotropes Hormon), Corticostatin, Calcitonin, Insulin, Oxytocin, Somatostatin und Analoga, LHRH-Analoga, Bombesin-Analoga, Cholecystokinin und Derivate, Endothelin und Analoga, Thrombin-Inhibitoren, Peptide Growth Factors (z.B. IGF, EGF, NGF), Magainine (PGS peptides), Gastrin-Analoga, Bradykinin-Analoga, Parathormon-Analoga, Neurokinin und Analoga, VIP und Analoga, ANP (Atriales natriuretisches Peptid) und Analoga, Neokyotrophin und Analoga, Angiotensin-Analoga, Enkephaline, Dynorphine, Dermorphine, Deltorphine, Renin-inhibierende Peptide, Tumor-Growth-Factor-Peptide, MSH (Melanocyte Stimulating Hormone)-Analoga, Mitotoxine, Tyrphostine, Chromogranin A, Thymopentin, TRH und Analoga, Substanz-P, Tuftsin, Fibronectin, und peptidische Immunmodulatoren wie Cyclosporin A, FK 506, Neuropeptid Y und NPK.

Erfindungsgemäß werden bevorzugt biotechnologisch hergestellte Peptide, insbesondere niedrige Peptide, verwendet.

Beispiele

1. Caproyldextrane

1.1 Herstellung

Für die Synthese wurden Dextrane mit Molmassen von 200 000 bis 300 000 und 1 000 000 bis 10 000 000 eingesetzt. Als Reaktionsmedium wurde Pyridin (inhomogene Quellungssynthese) oder Formamid (homogene Reaktionsmischung) verwendet. Als Acylierungsreagentien wurden die Säurechloride eingesetzt.

Im einzelnen wurde wie folgt verfahren:

Caproyldextran mit DS = 0,13 (Veresterungsgrad DS)

In einem 250-ml-Rundkolben mit Intensivkühler und Trockenrohr werden 4,0 g Dextran (Molmasse 1 000 000 bis 10 000 000) in 144 g Pyridin suspendiert. Es wird zwei Stunden bei 70°C gerührt, 2,1 g Capronsäurechlorid hinzugefügt und weitere drei Stunden gerührt. Das ausgefällte Produkt wird mehrfach mit Wasser und Aceton gewaschen.

Das Caproyldextran mit DS = 0,08 wurde analog hergestellt.

Caproyldextran mit DS = 1,7 (Vergleichsbeispiel)

In einem 250-ml-Rundkolben mit Intensivkühler und Trockenrohr werden 4,0 g Dextran in 136 g Pyridin suspendiert. Es wird zwei Stunden bei 70°C gerührt, 10,0 g Capronsäurechlorid hinzugefügt und weitere drei Stunden gerührt. Das ausgefällte Produkt wird mehrfach mit Wasser und Aceton gewaschen.

Das Caproyldextran mit DS = 0,62 wurde analog hergestellt.

1.2 Charakterisierung der Produkte

1.2.1 Substitutionsgradbestimmung

Der veresterten Caproylreste wurden nach alkalischer Hydrolyse als Capronsäuremethylester gaschromatographisch quantitativ bestimmt. Nach Auswertung der Peakflächen ergibt sich der Substitutionsgrad DS aus folgender Formel I

$$DS = \frac{Mol_{Cap} \cdot 162,14}{(Masse_{Po} - MG_{Cap} \cdot Mol_{Cap}) \cdot \left(1 + \frac{Mol_{Cap}}{MG_{Cap} \cdot Mol_{Cap}} \cdot MG_H\right)} \qquad I$$

Wobei die Gleichungsparameter folgende Bedeutung haben:

$Mol_{Cap} =$      mol Caproylsubstituent
$Masse_{Po} =$      Einwaage an Polymer
$MG_{Cap} =$      Molekulargewicht des Caproylsubstituenten
$MG_H =$      Molekulargewicht eines Wasserstoffatoms

1.2.2 Löslichkeit

Die wasserunlöslichen Capronsäureester, die aus Dextran der Molmasse 1 000 0.00 bis 10 000 000 gewonnen wurden, sind ausschließlich in Formamid und DMSO löslich.

Bei der niedriger gewählten Molmasse zeigen die gewonnenen Produkte, in Abhängigkeit des Substitutionsgrades, Löslichkeit in unterschiedlich polaren Lösungsmitteln.

| Molmasse | Substitutionsgrad | Methanol | Aceton | Formamid |
|---|---|---|---|---|
| 1.000.000- 10.000.000 | 0,08 | | | X |
| 1.000.000- 10.000.000 | 0,13 | | | X |
| 200.000- 300.000 | 0,62 | X | | |
| 200.000- 300.000 | 1,7 | | X | |
| Löslichkeit von Caproyldextranen | | | | |

### 1.2.3 Filmbildung

Die Caproyldextrane, die aus Dextran der Molmasse 200 000 - 300 000 gewonnen wurden, bilden stabile Filme aus den angegebenen Lösungsmitteln. Aus den höher molekularen Produkten konnten keine Filme gebildet werden.

| Molmasse | Substitutionsgrad | Filmbildung aus org. LM | Filmbildung aus wässr. Suspension | Stabilität der Filme in Wasser |
|---|---|---|---|---|
| 1.000.000- 10.000.000 | 0,08 | nein | schlecht | - |
| 1.000.000- 10.000.000 | 0,13 | nein | schlecht | - |
| 200.000- 300.000 | 0,62 | ja | nein | o |
| 200.000- 300.000 | 1,7 | ja | nein | + |
| Filmbildung und Filmstabilität der Caproyldextrane | | | | |

Wobei das Symbol - in der Spalte "Stabilität" einen Zerfall des Filmes in Wasser bedeutet, ein Symbol o einen Gewichtsverlust des Filmes bei der Quellung über 1% bedeutet und ein +-Symbol einen Gewichtsverlust des Filmes unter 1% bedeutet.

### 1.2.4 Wasseraufnahme der Caproyldextranfilme

Für einen enzymatischen Angriff ist es notwendig, daß die Filme im begrenzten Maße Wasser aufnehmen, da die Enzyme der Dickdarmflora im wäßrigen Medium in gelöster Form vorliegen. Während der Wasseraufnahme der Filme gelangen die Enzyme an die zu spaltenden Bindungen im Polymer.

Die Wasseraufnahme wird nach folgender Formel bestimmt.

$$A = \frac{G_t - G_o}{G_o} \cdot 100$$

Wobei A die Gewichtszunahme in Prozent, $G_o$ das Gewicht des trockenen Filmes und Gt das Gewicht des gequollenen, mit Wasser gesättigten Filmes bedeutet.

Folgende Werte wurden gefunden:

| Substitutionsgrad | 1,7 | 0,62 |
|---|---|---|
| Wasseraufnahme (%) | 3,3 | 37,5 |

### 1.2.5 Abbaubarkeit mit reiner Dextranase

Die Abbaubarkeit der Caproyldextrane wurde dünnschichtchromatographisch überprüft. Die niedermolekularen Substanzen wurden nicht enzymatisch angegriffen, da auch hier zu hoch substituiert werden muß, um wasserunlösliche Produkte zu erhalten.

Bei den höhermolekularen Capronsäureestern gelangt man schon mit einem niedrigen Substitutionsgrad oberhalb DS = 0,1 zu wasserunlöslichen Produkten. Bei dieser niedrigen Substitution bleibt die Abbaubarkeit erhalten.

### 1.3 Zusammenfassende Beurteilung der Caproyldextrane

Die Größen der Molmassen wurden zunächst gerade so gewählt, daß nach erfolgter Substitution ausreichend stabile Filme, im trockenen und gequollenen Zustand, gebildet werden können. Durch Einführung des C6-Substituenten wurde diese Anforderung bei einer Molmasse der Ausgangsdextrane zwischen 200.000 und 300.000 erfüllt. Bei dieser Molmasse führt ein Substitutionsgrad oberhalb von DS = 0,6 zu wasserunlöslichen Derivaten. Ein enzymatischer Angriff ist bei diesem Substitutionsgrad jedoch nicht mehr möglich.

Wird eine höhere Molmasse des Ausgangsdextrans für die Synthese, in einem Bereich zwischen 1 000 000 und 10 000 000 gewählt, so erhält man schon bei einem Substitutionsgrad oberhalb DS = 0,1 wasserunlösliche Produkte. Eine enzymatische Abbaubarkeit ist hier noch gewährleistet. Eine Filmbildung aus organischen Lösungsmitteln ist bei diesen hochmolekularen Derivaten aufgrund ihrer schlechten Löslichkeit nicht möglich. Die Thermogelierung aus wäßriger Suspension führte zwar zu Filmen, die jedoch keine ausreichende Stabilität in Wasser zeigen. Die Caproyldextrane mit den höheren Molmassen sind daher als Einbettungsmaterialien für eine Dickdarmapplikation geeignet.

| Molmasse | Substitutions-grad | löslich in | Filmbildung möglich aus LM/$H_2O$ | Quellung der Filme in Wasser | Abbaubarkeit durch Dextranase | Eignung als dickdarmabbaubarer Überzug |
|---|---|---|---|---|---|---|
| 1 Mio.-10 Mio | 0,08 | Formamid DMSO | nein/nein | - | ja | als Einbettung |
| 1 Mio.-10 Mio | 0,13 | Formamid DMSO | nein/ schlecht | zerfällt | ja | als Einbettung |

| Molmasse | Substitutions-grad | löslich in | Filmbildung möglich aus LM/$H_2O$ | Quellung der Filme in Wasser | Abbaubarkeit durch Dextranase | Eignung als dickdarmabbaubarer Überzug |
|---|---|---|---|---|---|---|
| 200 Tsd-300 Tsd | 0,62 | Methanol | ja/nein | 37,5 % | nein | nein |
| 200 Tsd-300 Tsd | 1,7 | Aceton | ja/nein | 3,3 | nein | nein |
| Charakterisierung von Caproyldextranen hinsichtlich ihrer Eignung als dickdarmabbaubarer Überzug | | | | | | |

2. Stearoyldextrane

2.1 Herstellung

Es wurde ein Dextran mit einer Molmasse zwischen 200 000 und 300 000 eingesetzt.

In der Literatur ist ein Verfahren zur Herstellung von Stearoyldextranen beschrieben (J. Chem. Soc., 1952, 74, 5016). Dabei wird die sog. Antreibermethode mit Chloracetanhydrid und Natriumperchlorat als Katalysator benutzt. Aus Stearinsäure wird während der Synthese durch Reaktion mit dem Antreiber das entsprechende Anhydrid gebildet. Dieses reagiert dann bevorzugt mit den Hydroxylgruppen des Dextrans. Chloracetylgruppen sind in den Reaktionsprodukten nicht nachweisbar. Als Lösungsmittel dient ein Überschuß an Chloracetanhydrid. Die Reaktion wird bei 80°C durchgeführt und läuft inhomogen ab. Durch die Zugabe unterschiedlicher Mengen Stearinsäure konnten Stearoyldextrane mit variablen Substitutionsgraden gewonnen werden.

Im einzelnen wurde wie folgt verfahren:

Stearoyldextran mit DS = 0,32

In einem 50 ml Rundkolben mit Intensivkühler werden 2,0 g Dextran, 4,0 g Stearinsäure, 20,0 g Chloracetanhydrid und 50 mg Natriumperchlorat eingewogen und unter Rühren auf 80°C erhitzt. Nach 8 Stunden wird die Reaktion abgebrochen und das ausgefällte Produkt mehrfach mit Wasser und Aceton gewaschen.

Das Stearoyldextran mit DS = 0,48 wurde analog hergestellt.

Stearoyldextran mit DS = 1,16 (Vergleichsbeispiel)

In einem 250 ml Rundkolben mit Intensivkühler und Trockenrohr werden 2,0 g Dextran in 140 g Pyridin suspendiert und bei 70°C zwei Stunden gerührt. Es wird 8,0 g Stearinsäurechlorid hinzugefügt und weitere vier Stunden gerührt. Das ausgefällte Produkt wird mehrfach mit Wasser und Aceton gewaschen.

2.2 Charakterisierung der Produkte

2.2.1 Substitutionsgradbestimmung

Die Stearoylsubstituenten werden durch alkalische Hydrolyse abgespalten, isoliert und nach Veresterung mit Methanol als Stearinsäuremethylester gaschromatographisch quantitativ bestimmt. Heptadecansäuremethylester dient für die Auswertung als innerer Standard.

Der Substitutionsgrad wurde analog zum Caproyldextran aus der Formel I berechnet.

2.2.2 Löslichkeit

Die erhaltenen Stearoylderivate sind lediglich in DMSO und Formamid löslich. Ein hochsubstituiertes, lipophiles Stearyldextran mit DS = 1,16 zeigte starke Quellung in Dichlormethan. Alle Produkte waren jedoch in Lösungsmitteln, aus denen ein Filmüberziehen von Drageekernen sinnvoll ist (z.B. Isopropanol), unlöslich.

| Substitutionsgrad | Formamid | DMSO | Dichlormethan |
|---|---|---|---|
| 0,32 | X | X | |
| 0,48 | X | X | |
| 1,16 | X | X | Quellung |
| Löslichkeit von Stearoyldextranen | | | |

### 2.2.3 Filmbildung

Filme können aus den gewonnenen Stearoyldextranen nur sehr schlecht gebildet werden. Das höher substituierte Derivat zeigte aus Dichlormethan Tendenz zur Filmbildung.

| Substitutionsgrad | Filmbildung aus org. LM | Filmbildung aus wäßriger Suspension | Stabilität der Filme in Wasser |
|---|---|---|---|
| 0,32 | nein | nein | |
| 0,48 | nein | nein | |
| 1,16 | schlecht | nein | o |
| Filmbildung und Filmstabilität der Stearoyldextranfilme | | | |

### 2.2.4 Wasseraufnahme des Stearoyldextranfilmes

Der erhaltene Film wurde in der gleichen Weise untersucht wie die Caproyldextranfilme.

Bei einem Substitutionsgrad von 1,16 wurde eine Wasseraufnahme von 3,75 % gefunden

### 2.2.5 Abbaubarkeit mit reiner Dextranase

Die Abbaubarkeit wurde dünnschichtchromatographisch überprüft. Die beiden niedersubstituierten Produkte werden enzymatisch angegriffen. Das Stearyldextran mit DS = 1,16 wird nicht abgebaut.

### 2.3 Zusammenfassende Beurteilung der Stearoyldextrane

Mit den lipophilen Stearinsäuresubstituenten können bei einer gewählten Molmasse zwischen 200 000 und 300 000 mit einem Substitutionsgrad unter DS = 0,5 wasserunlösliche Produkte erhalten werden, deren enzymatische Abbaubarkeit erhalten bleibt. Filme können aus diesen Produkten aufgrund ihrer schlechten Löslichkeitseigenschaften nicht gewonnen werden. Sie sind aber als Einbettungsmaterialien für eine gezielte Dickdarmapplikation geeignet. Das hoch substituierte Stearoylderivat besitzt zwar Tendenz zur Verfilmung, wird jedoch nicht abgebaut.

| Substitutionsgrad | löslich in | Filmbildung möglich aus LM/$H_2O$ | Quellung der Filme in Wasser | Abbaubarkeit durch Dextranase | Eignung als dickdarmabbaubarer Überzug |
|---|---|---|---|---|---|
| 0,32 | Formamid DMSO | nein/ nein | - | ja | als Einbettung |
| 0,48 | Formamid DMSO | nein/ nein | - | ja | als Einbettung |
| 1,16 | Quellung in Dichlormethan | ja/nein | 3,75 | nein | nein |
| Charakterisierung von Stearoyldextranen hinsichtlich ihrer Eignung als dickdarmabbaubarer Überzug | | | | | |

### 3. Lauroyldextrane

### 3.1 Herstellung

Es wurden Dextrane mit den Molmassenbereichen zwischen 200 000 und 300 000, 120 000 bis 170 000 und 60 000 bis 90 000 eingesetzt.

Die Synthese wird in Formamid, in dem Dextran sehr gut löslich ist, durchgeführt. Da Formamid, besonders unter dem Einfluß wasserziehender Mittel, bei erhöhter Temperatur zur Zersetzung neigt, wird die Synthese bei Raumtemperatur durchgeführt. Als Protonenfänger wurde Pyridin eingesetzt. Als Acylierungsmittel dient das entsprechende Säurechlorid. Laurinsäurechlorid bildet mit Formamid eine Art Gelkomplex, welcher zu erheblichen Viskositätsproblemen während der Synthese führen kann. Dies kann durch Zugabe überschüssiger Lösungsmittel behoben werden. Die Verwendung von Pyridin als Protonenfänger gegenüber anderer bei Raumtemperatur fester Basen wie z.B. 4-Dimethylaminopyridin erwies sich dabei als günstiger für die Senkung der Viskosität des Reaktionsansatzes.

Neben Pyridin wurde 4-Dimethylaminopyridin als Basenkatalysator eingesetzt. Dieses hatte den Vorteil, daß es gegenüber Pyridin besser aus dem Reaktionsprodukt durch Reinigung zu entfernen war. Die Synthese wird aus Gründen einer möglichen Formamidzersetzung unter dem Einfluß des Säurechlorides bei Raumtemperatur durchgeführt. Um mit dem vergleichsweise reaktionsträgen Laurinsäurechlorid kurze Reaktionszeiten von drei bis vier Stunden einzuhalten, wurde das Acylierungsmittel im großen Überschuß zugegeben. Dadurch wird der Reaktionsansatz trübe, was aber keinen Einfluß auf die Reproduzierbarkeit der Synthese hat. Es ist deshalb anzunehmen, daß Dextran und entstehende Dextranester während der Synthese weiterhin in Lösung bleiben und lediglich ein Teil des Acylierungsmittels ungelöst bleibt. Bei der Zugabe geringerer Mengen Säurechlorid bleibt der Ansatz homogen.

Im einzelnen wurde wie folgt verfahren:

Lauroyldextran mit DS = 0,08

In einem 250 ml Rundkolben mit Kühler und Trockenrohr werden 3,0 g Dextran und 2,2 g 4-Dimethylaminopyridin in 85 g Formamid gelöst und 9,6 g Laurinsäurechlorid hinzugefügt. Es wird 3,5 Stunden bei Raumtemperatur gerührt und die Reaktion durch Zugabe von Wasser abgebrochen. Das ausgefällte Produkt wird mehrfach mit einer Mischung aus Ethanol und Ethylacetat im Verhältnis 80:20 gewaschen.

Lauroyldextran mit DS = 0,11

In einem 250 ml Rundkolben mit Kühler und Trockenrohr werden 6,0 g Dextran in 90,0 g Formamid gelöst und 60,0 g Pyridin sowie 8,0 g Laurinsäurechlorid hinzugefügt. Es wird 3,5 Stunden bei Raumtemperatur gerührt und die Reaktion durch Zugabe von Wasser abgebrochen. Das ausgefällte Produkt wird mehrfach mit einer Mischung aus Ethanol und Ethylacetat im Verhältnis 80:20 gewaschen. Danach wird noch mehrfach mit Wasser gewaschen.

Lauroyldextran mit DS = 0,19

In einem 250 ml Rundkolben mit Kühler und Trockenrohr werden 3,0 g Dextran und 2,2 g 4-Dimethylaminopyridin in 81 g Formamid gelöst und 14,1 g Laurinsäurechlorid hinzugefügt. Es wird 3,5 Stunden bei Raumtemperatur gerührt und die Reaktion durch Zugabe von Wasser abgebrochen. Das ausgefällte Produkt wird mehrfach mit einer Mischung aus Ethanol und Ethylacetat im Verhältnis 80:20 gewaschen. Danach wird noch mehrfach mit Wasser gewaschen.

Die übrigen Lauroyldextrane wurden jeweils in analoger Weise hergestellt.

3.2 Charakterisierung der Produkte

3.2.1 Substitutionsgradbestimmung

Die Substituenten wurden nach Abspaltung Isolierung und Veresterung mit Methanol als Laurinsäuremethylester quantitativ gaschromatographisch bestimmt. Als innerer Standard dient Myristinsäuremethylester.

Der Substitutionsgrad DS wurde analog zum Caproyldextran aus der Formel I berechnet.

3.2.2 Löslichkeit

Bei einer Molmasse der Ausgangsdextrane zwischen 200 000 und 300 000 muß der Substitutionsgrad oberhalb von DS = 0,06 liegen, um wasserunlösliche Derivate zu erhalten. Wird eine kleinere Molmasse eingesetzt, muß etwas höher substituiert werden, um Produkte mit ähnlicher Löslichkeit zu gewinnen.

Lauroyldextrane mit Substitutionsgraden oberhalb dieser Grenzwerte sind in binären Lösungsmittelgemischen kolloidal löslich, wobei zum organischen Anteil (Isopropanol/Ethanol) stets Wasser zugesetzt werden muß. Die Opaleszenz geht bei Erhöhung der Temperatur verloren. Je nach Substitutionsgrad und Art des verwendeten organischen Anteils liegt der Klarpunkt zwischen 40°C und 60°C. Dies ist für das Auftragen der Filme auf Drageekerne aus warmen Lösungen für eine mögliche Thermolabilität von Wirk- oder Hilfsstoffen von Bedeutung.

| Molmasse | Substitutionsgrad | Wasser | Ethanol 50 % | Isopropanol 50% | Temperatur am Klarpunkt |
|---|---|---|---|---|---|
| 60.000- 90.000 | 0,12 | X | | | |
| 60.000- 90.000 | 0,30 | | X | | |

(fortgesetzt)

| Molmasse | Substitutionsgrad | Wasser | Ethanol 50 % | Isopropanol 50% | Temperatur am Klarpunkt |
|---|---|---|---|---|---|
| 120.000- 170.000 | 0,08 | X | | | |
| 120.000- 170.000 | 0,19 | | | X | 40°C |
| 120.000- 170.000 | 0,28 | | | X | |
| 200.000- 300.000 | 0,06 | X | | | |
| 200.000- 300.000 | 0,11 | | | X | 55°C |
| 200.000- 300.000 | 0,24 | | | X | |
| Löslichkeit von Lauroyldextranen | | | | | |

### 3.2.3 Filmbildung

Eine Tendenz zur Filmbildung zeigt sich bei einer Molmasse oberhalb 120 000. Qualitativ gute Filme erhält man bei Derivaten mit einer Molmasse oberhalb 200 000. Die Filme konnten sowohl aus kalten bzw. warmen Lösungen, als auch aus wäßrigen Suspensionen bei 37°C gewonnen werden. Um homogene Filme mittels Thermogelierung zu erhalten, sollte die Partikelgröße in der Suspension bei der Filmgewinnung im gequollenen Zustand unter 30 µm liegen.

Die Filme, die aus Derivaten der Molmasse zwischen 120 000 und 170 000 gewonnen wurden zerfallen in Wasser innerhalb eines Zeitraums von 30 bis 120 Minuten, obwohl es sich bei diesen Produkten um wasserunlösliche Derivate handelt.

| Molmasse | Substitutionsgrad | Filmbildung aus org. LM | Filmbildung aus wässr. Suspension | Stabilität der Filme in Wasser |
|---|---|---|---|---|
| 60.000- 90.000 | 0,30 | nein | nein | - |
| 120.000- 170.000 | 0,19 | ja | ja | Zerfall nach 30 min |
| 120.000- 170.000 | 0,28 | ja | ja | Zerfall nach 100 min |
| 200.000- 300.000 | 0,11 | ja | ja | + |
| 200.000- 300.000 | 0,24 | ja | ja | + |
| Filmbildung der Lauroyldextrane | | | | |

### 3.2.4 Wasseraufnahme der Filme

Die Wasseraufnahme wurde nach beschriebener Methode bestimmt.

Folgende Werte wurden gefunden:

| Substitutionsgrad | 0,28* | 0,11** | 0,24** |
|---|---|---|---|
| Wasseraufnahme | 198 | 230 | 178 |

\*: Dextran 150

\*\*: Dextran 250

### 3.2.5 Abbaubarkeit mit reiner Dextranase

Alle erhaltenen Produkte sind enzymatisch abbaubar. Nach abgeschlossenem enzymatischen Angriff verbleibt bei einigen wasserunlöslichen Produkten in Abhängigkeit vom Substitutionsgrad ein wasserunlöslicher Rest. Lauroyldextran mit DS = 0,11 wird zu vollständig wasserlöslichen Abbauprodukten gespalten. Die Löslichkeitsgrenze in Wasser liegt bei einer Molmasse von ca. 60 000.

### 3.3 Zusammenfassende Beurteilung der Lauroyldextrane

Durch die Substitution mit Laurinsäure können Dextranderivate gewonnen werden, die den Anforderungen an einen filmbildenden, dickdarmabbaubaren Überzug in allen Belangen entsprechen. Eine Wasserunlöslichkeit ist bei einer Molmasse zwischen 60 000 und 90 000 des eingesetzten Dextrans oberhalb DS = 0,12, zwischen 120 000 und 170 000 oberhalb DS = 0,08 und zwischen 200 000 und 300 000 oberhalb DS = 0,06 gegeben. Für die Stabilitat der Filme in Wasser ist ein Substitutionsgrad zwischen DS = 0,1 und 0,2 bei einer für die Filmbildung erforderlichen Molmasse zwischen 200 000 und 300 000 notwendig. Die Produkte sind in Ethanol 50 % bzw. Isopropanol 50 % kolloidal löslich. Bei Erhöhung der Temperatur entstehen klare Polymerlösungen. Filme können aus solchen Lösungen gewonnen werden. Eine Thermogelierung aus wäßriger Suspension ist ebenfalls möglich. Damit Lauroyldextranfilme mit ausreichender Stabilität in Wasser gebildet werden können, muß die Molmasse der Ausgangsdextrane oberhalb 200 000 liegen.

Es konnten somit zwei Substanzen hergestellt werden, die besonders geeignet sind. Das Derivat mit DS = 0,11 besitzt gegenüber dem mit DS = 0,24 jedoch zusätzlich bessere Abbaueigenschaften, da vollständig wasserlösliche Abbauprodukte entstehen. Eine Arzneistofffreisetzung ist somit einerseits durch Nachlassen der mechanischen Festigkeit der Filme gegeben, andererseits durch Auflösen des Filmes sicher gewährleistet.

| Molmasse | Substitutionsgrad | löslich in | Filmbildung möglich aus LM/H$_2$O | Wasseraufnahme der Filme | Abbau durch Dextranase möglich | Eignung als dickdarmabbaubarer Überzug |
|---|---|---|---|---|---|---|
| 120.000- 170.000 | 0,28 | Isopropanol 50% | ja/ja | 198 % | ja | evtl. als Einbettung Film zerfällt |
| 200.000- 300.000 | 0,11 | Isopropanol 50% | ja/ja | 230 % | ja | ja |
| 200.000- 300.000 | 0,24 | Isopropanol | ja/ja | 178 % | ja | ja |
| Charakterisierung der Lauroyldextrane hinsichtlich Eignung als dickdarmabbaubare Überzüge | | | | | | |

4. Vergleichsversuch mit Acetyldextranen

Als Vergleichssubstanzen wurden einige Acetyldextrane hergestellt. Es wurden Dextrane mit einer Molmasse von 1 000 000 bis 10 000 000 eingesetzt.

Die Herstellung erfolgte wie in der Literatur (J. Am. Chem. Soc. 74, 5339, 1952) beschrieben.

Im einzelnen wurde z.B. wie folgt verfahren:

Acetyldextran mit DS = 3

In einem 250 ml Rundkolben mit Intensivkühler und Trockenrohr werden 3,0 g Dextran mit 122,6 g Formamid gelöst und anschließend 10,5 g Pyridin und soviel Acetanhydrid hinzugefügt, daß das molare Verhältnis von reaktiven Acetylgruppen zu freien Hydroxylgruppen des Dextrans 1,2:1 beträgt. Es wird drei Stunden bei Raumtemperatur gerührt. Die Reaktion wird durch Zugabe von Wasser abgebrochen. Das Reaktionsprodukt wird ausgefällt und mehrmals mit Wasser gewaschen. Die anderen Acetyldextrane wurden auf analoge Weise hergestellt.

Die Charakterisierung erfolgte wie bei den anderen Dextranestern beschrieben. Zwar konnten filmbildende Verbindungen erhalten werden, aber es wurde festgestellt, daß unterhalb eines Substitutionsgrades von DS = 1,2 Acetyldextran wasserlöslich und damit für die erfindungsgemäßen Zwecke nicht geeignet ist. Wasserunlösliche Acetyldextrane mit DS > 1,2 werden nicht mehr abgebaut und sind daher ebenfalls nicht geeignet (s. untenstehende Tabelle).

| Substitutionsgrad DS | löslich in | Filmbildung möglich aus LM/$H_2O$ | Wasseraufnahme der Filme | Abbau durch Dextranase möglich | Eignung als dickdarmabbaubarer Überzug |
|---|---|---|---|---|---|
| 3 | Tetrachlorethan | ja/nein | 1,8 % | nein | nein |
| 2,4 | Dichlormethan | ja/nein | 9,5 % | nein | nein |
| 1,84 | Aceton | ja/nein | 58,2 % | nein | nein |
| 1,4 | Methanol | ja/nein | 135,5 % | nein | nein |
| Charakterisierung von Acetyldextranen mit unterschiedlichen Substitutionsgraden hinsichtlich der Eignung als dickdarmabbaubarer Überzug. | | | | | |

5. Analytische Methoden

5.1 Bestimmung des Substitutionsgrads von Caproyldextran

50,0 mg Caproyldextran und 20,0 mg Heptansäuremethylester werden in einem Vial mit 5 ml KOH 10% versetzt, zugebördelt und 3 Stunden bei 90°C temperiert. Nach dem Abkühlen wird die Lösung in einen 50 ml Scheidetrichter überführt, mit HCl konz. angesäuert und dreimal mit je 10 ml Diethylether ausgeschüttelt. Die über Natriumsulfat getrockneten Etherphasen werden in einen 50 ml Rundkolben überführt und der Ether am Rotationsverdampfer abgezogen. Zum Rückstand gibt man 10,0 ml Methanol und 5,0 ml methanolische Bortrifluoridlösung 50 % und erhitzt unter Rückflußkühlung 30 Minuten zum Sieden. Die Reaktion wird durch Zugabe von 5 ml Wasser abgebrochen und das abgekühlte Reaktionsgemisch dreimal mit je 5 ml Hexan ausgeschüttelt. Die getrockneten Hexanphasen werden vereinigt und dienen als Einspritzlösung für die Gaschromatographie.

Zur Faktorbestimmung werden jeweils 20,0 mg Capronsäuremethylester und Heptansäuremethylester in 5,0 ml Hexan gelöst und eingespritzt. Die Peakflächen werden gegeneinander ausgewertet.

Gaschromatographie:

Säule: DEGS
Ofentemperatur: 70°C
Einspritztemperatur: 100°C
Einspritzvolumen: 1 µl

Die Substitutionsgrade der anderen Dextranester wurden in analoger Weise bestimmt (Berechnung mit obiger Formel I).

5.2 Gewinnung der Filme

5.2.1 Aus organischen Lösungsmitteln

100 mg Dextranester werden in 2 ml eines geeigneten Lösungsmittel gelöst und in eine Teflonschale mit 3 cm Durchmesser ausgegossen. Das Lösungsmittel läßt man bei 37°C abdampfen.

5.2.2 Thermogelierung

200 mg Dextranester werden in 2 ml Wasser suspendiert. Nach halbstündiger Quellung des Polymers wird die Suspension mit einem Ultraturrax 5 Minuten dispergiert. Die Suspension wird in eine Teflonschale mit 3 cm Durchmesser gegossen. Der Film wird durch Verdunsten des Wasser bei 37°C gewonnen.

5.3 Wasseraufnahme der Filme

10 mg eines homogenen Films werden in 5 ml Wasser eingelegt. Nach vollständiger Quellung wird Oberflächenwasser mit Filterpapier von der Filmoberfläche entfernt und der Film erneut gewogen. Die Wasseraufnahme wird prozentual angegeben.

5.4 Abbaubarkeit der Dextranester

Untersuchung der Abbaubarkeit mit Hilfe der Dünnschichtchromatographie

50 mg Dextranester werden in 5 ml Phosphatpuffer pH 6,8 suspendiert. Nach einer Stunde gibt man 1 ml einer Dextranaselösung hinzu, die 6 U/ml Enzym enthält. Die Mischung wird eine Stunde bei 37°C inkubiert und die enzymatische Reaktion durch Zugabe von 100 µl Methanol abgebrochen. Als Auftragslösung dient der klare Überstand. 50 mg unsubstituiertes Dextran wird auf die gleiche Weise behandelt und dient als Blindwert.

Dünnschichtchromatographie:

| | |
|---|---|
| Auftragsvolumen: | 20 µl |
| Bandenbreite: | 15 mm |
| Laufstrecke: | 15 cm |
| Laufmittel: | 1-Propanol:Butanol:Nitromethan:Wasser im Verhältnis 4:1:2:3 |
| Vergleiche: | 0,1 %ige Lösungen von Glucose, Isomaltose, Isomaltotriose |
| Detektion: | Eckerts-Regenz/120°C |

Zusammenfassend ist festzustellen, daß es durch Variation von Substituent, Substitutionsgrad und Molmasse des Ausgangsdextrans gelang, gezielt Derivate darzustellen, die allen Anforderungen bezüglich ihrer Eignung als dickdarmabbaubare Überzüge entsprechen. Es wurden Molmassen im Bereich zwischen 60 000 und 10 000 000 eingesetzt. Bei der Substitution mit $C_6$- und $C_{12}$-Fettsauren liegt die Molmasse des Dextrans bevorzugt oberhalb 200 000, damit aus den Produkten mechanisch stabile Filme im trockenen und gequollenen Zustand gewonnen werden können. Eine Tendenz zur Filmbildung ist schon bei einer Molmasse von ca. 120 000 zu erkennen. Die Stabilität solcher Filme ist aber speziell im gequollenen Zustand gering. Aus Stearoyldextranen konnten nur ungenügende Filme gebildet werden. Für einen möglichen enzymatischen Angriff müssen größere unsubstituierte Bereiche im Polymer vorliegen. Unterhalb eines Substitutionsgrades von bevorzugt DS = 0,5 bleibt jedenfalls eine Abbaubarkeit erhalten. Mit geeigneten Substituenten wurden deshalb bei diesem optimal ermittelten Substitutionsgrad wasserunlösliche Derivate erhalten. Mit der Einführung von Lauroylsubstituenten konnten Derivate erhalten werden, die allen Anforderungen entsprechen. Stabile Filme, im trockenen und gequollenen Zustand, erhalt man bei einer Molmasse oberhalb 200 000 und einem Substitutionsgrad oberhalb von DS = 0,1. Filme können aus organischen Lösungsmitteln, z.B. Isopropanol 50 %, oder mittels Thermogelierung bei 37°C gewonnen werden. Bei diesem geringen Substitutionsgrad ist die Abbaubarkeit des Produktes gewährleistet.

**Patentansprüche**

1. Dextranester mit einer Molmasse von 40 000 bis 10 000 000 und Esterseitenketten, die sich von Säuren mit 6 bis 18 C-Atomen ableiten, wobei der Veresterungsgrad in Abhängigkeit von der C-Atomzahl der Seitenketten und der Molmasse auf einen Wert von 0,04 bis 1,1 so eingestellt wird, daß die Dextranester bei Raumtemperatur in Wasser unlöslich sind und von Dickdarmbakterien abgebaut werden.

2. Dextranester nach Anspruch 1, dadurch gekennzeichnet, daß die Esterseitenketten von Säuren mit 8 bis 16 C-Atomen abgeleitet sind, die Molmasse 40 000 bis 1 000 000 beträgt und der Veresterungsgrad bei 0,08 bis 0,8 liegt.

3. Dextranester nach Anspruch 2, dadurch gekennzeichnet, daß die Esterseitenketten von Säuren mit 8 bis 12 C-Atomen abgeleitet sind, die Molmasse 60 000 bis 400 000 beträgt und der Veresterungsgrad bei 0,1 bis 0,5 liegt.

4. Verwendung der Dextranester nach mindestens einem der Ansprüche 1 bis 3 zur Umhüllung und/oder Einbettung von Arzneiwirkstoffen oder Arzneimittelzubereitungen.

# EP 0 719 286 B1

5. Verfahren zur Herstellung der Dextranester nach Anspruch 1, dadurch gekennzeichnet, daß Dextran in einem Lösungsmittel aus Formamid und/oder Dimethylsulfoxid, dem eine derartige Menge eines aprotischen, polaren, organischen Solvens zugemischt werden kann, daß das Dextran noch löslich ist, gelöst wird und ein Halogenid einer Säure mit 6 bis 18 C-Atomen in Gegenwart eines Protonenfängers so zugegeben wird, daß die Temperatur des Reaktionsgemisches nicht über 40°C steigt.

6. Arzneimittel, dadurch gekennzeichnet, daß es einen im Dickdarm wirkenden Wirkstoff oder einen Wirkstoff, der beim Durchgang durch den Magen oder Dünndarm abgebaut wird, umhüllt mit oder eingebettet in einem Dextranester nach einem der Ansprüche 1 bis 3 enthält.

7. Arzneimittel nach Anspruch 6, dadurch gekennzeichnet, daß es als Wirkstoff ein Peptidarzneimittel enthält.

8. Arzneimittel nach Anspruch 6 oder 7, dadurch gekennzeichnet, daß es in Form von Tabletten, einem Granulat oder Kapseln vorliegt.

## Claims

1. A dextran ester with a molecular weight of 40,000-10,000,000 and ester side chains which are derived from acids with 6-18 carbon atoms, where the degree of esterification is adjusted, depending on the number of carbon atoms in the side chains and the molecular weight, to a value of 0.04-1.1 so that the dextran ester is insoluble in water at room temperature and is broken down by colonic bacteria.

2. A dextran ester as claimed in claim 1, wherein the ester side chains are derived from acids with 8-16 carbon atoms, the molecular weight is 40,000-1,000,000, and the degree of esterification is 0.08-0.8.

3. A dextran ester as claimed in claim 2, wherein the ester side chains are derived from acids with 8-12 carbon atoms, the molecular weight is 60,000-400,000, and the degree of esterification is 0.1-0.5.

4. The use of the dextran esters as claimed in at least one of claims 1 to 3 for coating and/or embedding pharmaceutical active ingredients or drug formulations.

5. A process for preparing the dextran ester as claimed in claim 1, which comprises dissolving dextran in a solvent composed of formamide and/or dimethyl sulfoxide, to which it is possible to add an amount of an aprotic polar organic solvent such that the dextran is still soluble, and adding a halide of an acid with 6-18 carbon atoms in the presence of a proton scavenger in such a way that the temperature of the reaction mixture does not exceed 40°C.

6. A drug which contains an active ingredient which acts in the colon or an active ingredient which is broken down on passing through the stomach or small intestine, coated with or embedded in a dextran ester as claimed in any of claims 1 to 3.

7. A drug as claimed in claim 6, which contains a peptide as active ingredient.

8. A drug as claimed in claim 6 or 7, which is in the form of tablets, granules or capsules.

## Revendications

1. Esters du dextrane d'une masse molaire de 40 000 à 10 000 000 et à chaînes latérales du type ester, qui proviennent d'acides comportant de 6 à 18 atomes de carbone, où le degré d'estérification est réglé en dépendance du nombre d'atomes de carbone des chaînes latérales et de la masse molaire, à une valeur de 0,04 à 1,1, en une manière telle que les esters du dextrane soient insolubles dans l'eau à la température ambiante et soient dégradés par les bactéries du gros intestin.

2. Esters du dextrane suivant la revendication 1, caractérisés en ce que les chaînes latérales du type ester proviennent d'acides qui comportent de 8 à 16 atomes de carbone, dont la masse molaires varie de 40 000 à 1 000 000 et dont le degré d'estérification se situe dans la plage de 0,08 à 0,8.

3.  Esters du dextrane suivant la revendication 2, caractérisés en ce que les chaînes latérales du type ester dérivent d'acides qui comportent de 8 à 12 atomes de carbone, dont la masse molaire varie de 60 000 à 400 000 et dont le degré d'estérification varie dans la plage de 0,1 à 0,5.

4.  Utilisation des esters du dextrane suivant au moins l'une des revendications 1 à 3, en vue de l'enrobage et/ou de l'incorporation de médicaments ou de préparations de médicaments.

5.  Procédé de préparation des esters du dextrane suivant la revendication 1, caractérisé en ce que l'on dissout le dextrane dans un solvant constitué de formamide et/ou de sulfoxyde de diméthyle, auquel on a mélangé une proportion d'un solvant organique, polaire et aprotique, en une manière telle que le dextrane soit encore soluble et on ajoute un halogénure d'un acide qui comporte de 6 à 18 atomes de carbone, en proportion d'un récepteur de protons, en une manière telle que la température du mélange réactionnel ne s'élève pas au dessus de 40°C.

6.  Médicament, caractérisé en ce qu'il contient un principe actif agissant dans le gros intestin, ou un principe actif qui est dégradé par le passage à travers l'estomac ou l'intestin grêle, enrobé par ou noyé dans un ester du dextrane suivant l'une quelconque des revendications 1 à 3.

7.  Médicament suivant la revendication 6, caractérisé en ce qu'il contient un médicament peptidique, à titre de principe actif.

8.  Médicament suivant la revendication 6 ou 7, caractérisé en ce qu'il se présente sous la forme de comprimés, d'un granulé ou de gélules.